(19) Europäisches Patentamt
European Patent Office
Office européen des brevets

(11) Veröffentlichungsnummer: **0 130 517**
**A2**

# (12) EUROPÄISCHE PATENTANMELDUNG

(21) Anmeldenummer: **84107250.7**

(22) Anmeldetag: **25.06.84**

(51) Int. Cl.⁴: **C 07 D 253/06**, A 01 N 43/707
// C07C121/76, C07C49/233

(30) Priorität: **02.07.83 DE 3323934**

(43) Veröffentlichungstag der Anmeldung: **09.01.85 Patentblatt 85/2**

(84) Benannte Vertragsstaaten: **BE CH DE FR GB IT LI NL**

(71) Anmelder: **BAYER AG, Konzernverwaltung RP Patentabteilung, D-5090 Leverkusen 1 Bayerwerk (DE)**

(72) Erfinder: **Kranz, Eckart, Dr., Am Acker 9, D-5600 Wuppertal 1 (DE)**
Erfinder: **Findeisen, Kurt, Dr., In der Follmuehle 10, D-5068 Odenthal 2 (DE)**
Erfinder: **Fiege, Helmut, Dr., Walter-Flex-Strasse 23, D-5090 Leverkusen 1 (DE)**
Erfinder: **Eue, Ludwig, Dr., Paul-Klee-Strasse 36, D-5090 Leverkusen 1 (DE)**
Erfinder: **Schmidt, Robert R., Dr., Im Waldwinkel 110, D-5060 Bergisch Gladbach 2 (DE)**

## (54) 3,6-Disubstituierte 4-Amino-1,2,4-triazin-5-one.

(57) Neue 3,6-disubstituierte 4-Amino-1,2,4-triazin-5-one der allgemeinen Formel (I)

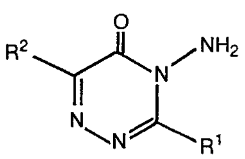

(I)

in welcher
$R^1$ für Alkylthio, Alkylamino oder Dialkylamino steht und
$R^2$ für die Gruppierungen

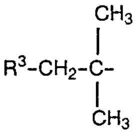 ; 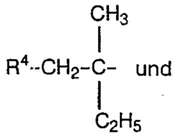 und 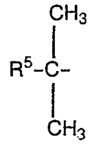 steht, wobei

$R^3$ für gegebenenfalls substituiertes Phenyl, gegebenenfalls substituiertes Phenoxy oder Alkylcarbonyloxy steht,
$R^4$ für Alkoxy steht und
$R^5$ für Dihalogenmethyl steht,

ein Verfahren zu ihrer Herstellung sowie ihre Verwendung als Herbizide.

EP 0 130 517 A2

0130517

BAYER AKTIENGESELLSCHAFT
Konzernverwaltung RP,
Patentabteilung

5090 Leverkusen, Bayerwerk

Bi/by
Ia

3,6-Disubstituierte 4-Amino-1,2,4-triazin-5-one

Die vorliegende Erfindung betrifft neue 3,6-disubstituierte 4-Amino-1,2,4-triazin-5-one, ein Verfahren zu ihrer Herstellung sowie ihre Verwendung als Herbizide, insbesondere als selektive Herbizide.

Es ist bereits bekannt geworden, daß bestimmte substituierte 1,2,4-Triazin-5-on-Derivate, wie beispielsweise 4-Amino-6-fluor-tert.-butyl-3-methylthio-1,2,4-triazin-5-on, als Herbizide verwendet werden können (vgl. EP-A 0 049 416 /Le A 20 631/). In bestimmten Kulturen ist jedoch ein selektiver Einsatz der vorbekannten Triazinone nicht möglich, da es infolge der durchweg hohen herbiziden Potenz dieser Stoffgruppe auch bei bestimmten Nutzpflanzen zu Schäden kommen kann; die Verträglichkeit gegenüber den vorbekannten Triazinonen ist demnach bei verschiedenen Nutzpflanzen nicht ausreichend.

Es wurden neue 3,6-disubstituierte 4-Amino-1,2,4-triazin-5-one der allgemeinen Formel (I),

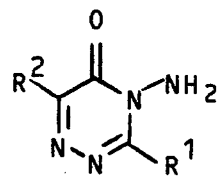

(I)

in welcher

$R^1$ für Alkylthio, Alkylamino oder Dialkylamino steht und

$R^2$ für die Gruppierungen

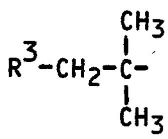 ; 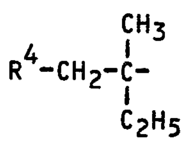 und 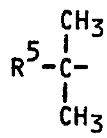 steht, wobei

$R^3$ für gegebenenfalls substituiertes Phenyl, gegebenenfalls substituiertes Phenoxy oder Alkylcarbonyloxy steht,

$R^4$ für Alkoxy steht und

$R^5$ für Dihalogenmethyl steht,

aufgefunden.

Weiterhin wurde gefunden, daß man die 3,6-disubstituierten 4-Amino-1,2,4-triazin-5-one der Formel (I) erhält, wenn man in einer <u>ersten Stufe</u> Cyanide der Formel (II),

$$R^2\text{-CO-CN} \qquad (II)$$

in welcher

$R^2$ die oben angegebene Bedeutung hat,

a) gegebenenfalls in Gegenwart einer flüssigen Carbonsäure als Lösungsmittel, mit einer anorganischen Säure umsetzt oder

b) an ein Carboniumion addiert, das aus einem Olefin, wie z.B. Isobutylen, oder einem tertiären Alkohol, wie z.B. tert.-Butanol, mit einer starken Säure, wie insbesondere Schwefelsäure, gebildet wird, und anschließend der Hydrolyse unterwirft (sog. RITTER-REAKTION)

und

die hierbei entstehenden Ketocarbonsäureamide der Formel (IIIa),

$$R^2\text{-CO-CO-NHR} \qquad \text{(IIIa)}$$

in welcher

$R^2$ die oben angegebene Bedeutung hat und

R für Wasserstoff (erhältlich nach Variante a) oder Alkyl, insbesondere tert.-Butyl (erhältlich nach Variante b) steht,

in einer <u>zweiten Stufe</u> in an sich bekannter Weise entweder direkt in der anfallenden Lösung oder nach Zwischenisolierung, gegebenenfalls nach vorheriger Verseifung zu den freien Ketocarbonsäuren der Formel (IIIb),

$$R^2\text{-CO-COOH} \qquad \text{(IIIb)}$$

in welcher

$R^2$ die oben angegebene Bedeutung hat,

mit Thiocarbohydrazid der Formel (IV),

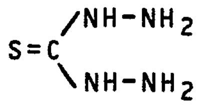

(IV)

in wässriger bzw. wässrig-saurer Lösung, gegebenenfalls in Gegenwart eines organischen Verdünnungsmittels, zu 4-Amino-3-mercapto-1,2,4-triazin-5-onen der Formel (V),

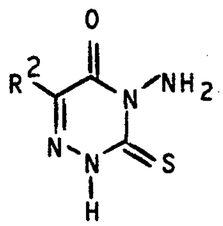

(V)

in welcher

$R^2$ die oben angegebene Bedeutung hat,

umsetzt; diese in einer dritten Stufe in bekannter Weise in alkalischer Lösung mittels Alkylhalogenid, vorzugsweise Alkyljodid oder -bromid, zu 3-Alkylthio-4-amino-1,2,4-triazin-5-onen der Formel (Ia),

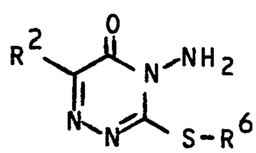

(Ia)

in welcher

$R^2$ die oben angegebene Bedeutung hat und

$R^6$ für Alkyl, vorzugsweise mit 1 bis 4 Kohlenstoffatomen, steht,

alkyliert, diese gegebenenfalls in einer vierten Stufe mit Aminen der Formel (VI),

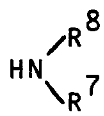

(VI)

in welcher

$R^8$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, steht,

in Gegenwart eines Verdünnungsmittels umsetzt.

Außerdem wurde gefunden, daß die 3,6-disubstituierten 4-Amino-1,2,4-triazin-5-one der Formel (I) gute herbizide, insbesondere selektiv-herbizide Eigenschaften aufweisen.

Ueberraschenderweise zeigen die erfindungsgemäßen Verbindungen gegenüber dem bekannten 4-Amino-6-fluor-tert.-butyl-3-methylthio-1,2,4-triazin-5-on, welches konstitutionell und wirkungsmäßig eine naheliegende Verbindung ist, bei einer vergleichbar guten allgemeinen herbiziden Wirkung, insbesondere eine bessere Verträglichkeit bei wichtigen Kulturpflanzen, wie bei Hafer, Mais, Weizen und anderen. Die erfindungsgemäßen Wirkstoffe stellen somit eine Bereicherung der herbiziden Mittel, insbesondere der selektiven chemischen Unkrautbekämpfung dar.

Die erfindungsgemäßen 3,6-disubstituierten 4-Amino-1,2,4-triazin-5-one sind durch die Formel (I) allgemein definiert. In dieser Formel stehen vorzugsweise

$R^1$ für geradkettiges oder verzweigtes Alkylthio mit 1 bis 4 Kohlenstoffatomen, sowie für Alkylamino und Dialkylamino mit jeweils 1 bis 6 Kohlenstoffatomen in jedem Alkylteil;

$R^2$ für die Gruppierungen

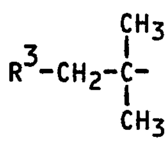 ; 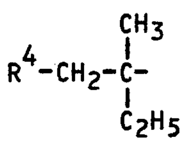 und 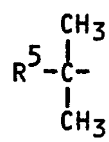 ;

$R^3$ für Alkylcarbonyloxy mit 1 bis 4 Kohlenstoffatomen im Alkylteil, sowie für jeweils gegebenenfalls einfach bis dreifach, gleich oder verschieden substituiertes Phenyl und Phenoxy, wobei als Substituenten genannt seien :
Halogen, Alkyl mit 1 bis 2 Kohlenstoffatomen, Nitro und Halogenalkyl mit 1 bis 2 Kohlenstoffatomen und 1 bis 5 gleichen oder verschiedenen Halogenatomen, wie Fluor- und Chloratomen;

$R^4$ für Alkoxy mit 1 bis 4 Kohlenstoffatomen und

$R^5$ für Dihalogenmethyl mit Fluor und Chlor als Halogenatome.

Besonders bevorzugt sind diejenigen Verbindungen der Formel (I), in denen

$R^1$ für Methyl-, Ethyl- oder Propylthio; für Methyl-, Ethyl-, Propyl- oder Hexylamino; sowie für Dimethyl-, Diethyl- oder Ethylmethylamino steht;

$R^2$ für die Gruppierungen

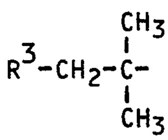 , 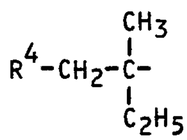 und 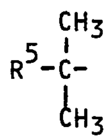 steht;

$R^3$ für Methylcarbonyloxy oder Ethylcarbonyloxy; sowie für jeweils gegebenenfalls einfach bis dreifach, gleich oder verschieden substituiertes Phenyl oder Phenoxy steht, wobei als Substituenten genannt seien :
Fluor, Chlor, Methyl, Nitro und Trifluormethyl;

$R^4$ für Methoxy, Ethoxy oder Isopropoxy steht und

$R^5$ für Dichlormethyl steht.

Verwendet man beispielsweise Phenylpivaloylcyanid als Ausgangsstoff in der ersten Stufe, setzt in der zweiten Stufe die entsprechende freie Säure mit Thiocarbohydrazid um und verwendet in der dritten Stufe Methylbromid als Alkylierungsmittel, so kann der Reaktionsablauf durch das folgende Formelschema wiedergegeben werden :

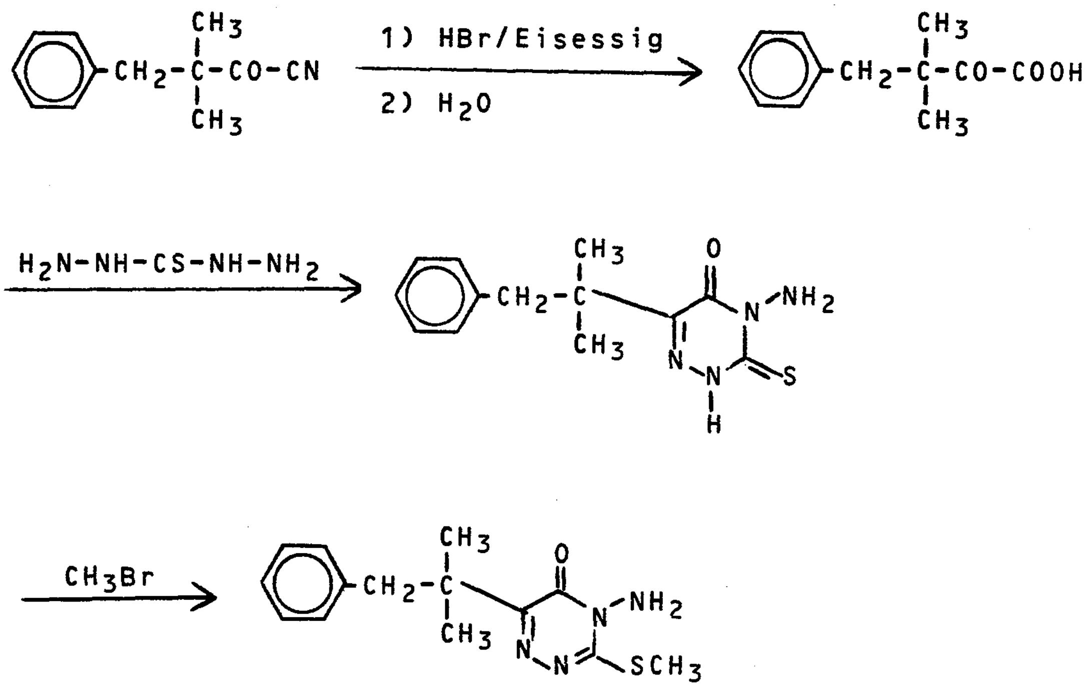


Verwendet man das so erhaltene 4-Amino-3-methylthio-6-phenyl-tert.-butyl-1,2,4-triazin-5-on und Dimethylamin als Ausgangsstoffe für die vierte Stufe, so kann der Reaktionsablauf durch das folgende Formelschema wiedergegeben werden :

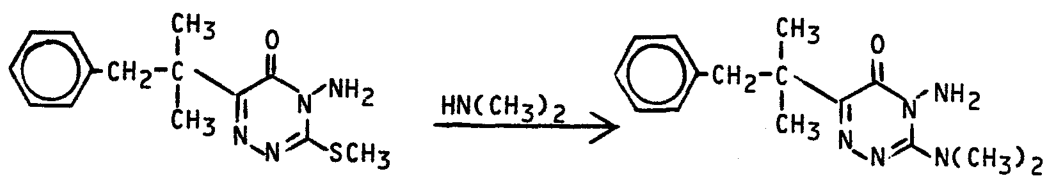

Die für das erfindungsgemäße Verfahren als Ausgangsstoffe zu verwendenden Cyanide sind durch die Formel (II) allgemein definiert. In dieser Formel steht $R^2$ vorzugsweise für die Reste, die bereits im Zusammenhang mit der Beschreibung der erfindungsgemäßen Stoffe der Formel (I) vorzugsweise für diesen Substituenten genannt wurden.

Die Cyanide der Formel (II) sind noch nicht bekannt, sie können jedoch in bekannter Art und Weise erhalten werden, indem man z.B. entsprechende Halogenide mit Trimethylsilylcyanid gegebenenfalls in Gegenwart eines Verdünnungsmittels umsetzt. Trimethylsilylcyanid, $(CH_3)_3$-Si-CN, ist bekannt (vgl. z.B. Synthesis, 1979, S. 522 und 523).

Das Thiocarbohydrazid der Formel (IV) und die Amine der Formel (VI) sind allgemein bekannte Verbindungen der organischen Chemie.

Die erste Stufe des erfindungsgemäßen Verfahrens kann in Abwesenheit oder in Gegenwart einer unter den Reaktionsbedingungen flüssigen, niederaliphatischen Carbonsäure als Lösungsmittel durchgeführt werden. Als solche Lösungsmittel kommen vorzugsweise Carbonsäuren mit 1 bis 6 Kohlenstoffatomen, wie Essigsäure, Propionsäure oder Ameisensäure, in Frage.

Die erste Stufe des erfindungsgemäßen Verfahrens wird mit Hilfe einer starken anorganischen Säure durchgeführt. Hierzu gehören vorzugsweise die Halogenwasserstoffsäuren, wie Chlorwasserstoff und Bromwasserstoff, sowie konzentrierte Schwefelsäure.

Die Reaktionstemperaturen können bei der Durchführung dieser Verfahrensstufe in einem größeren Bereich variiert werden. Im allgemeinen arbeitet man zwischen -20°C und +50°C, vorzugsweise zwischen 0°C und +40°C.

Bei der Durchführung der ersten Stufe des erfindungsgemäßen Verfahrens setzt man auf 1 Mol Cyanid der Formel (II) vorzugsweise 1 bis 10 Mol an anorganischer Säure ein.

Die zweite Stufe des erfindungsgemäßen Verfahrens erfolgt vorzugsweise ohne vorherige Zwischenisolierung der α-Ketocarbonsäureamide der Formel (IIIa) und nach Verseifung derselben in üblicher Weise zu freien Säuren der Formel (IIIb).

Die zweite Stufe des erfindungsgemäßen Verfahrens wird in wäßriger Lösung bzw. in Gegenwart einer wäßrig-sauren Lösung wie einer halogenwasserstoffsauren, vorzugsweise einer salzsauren, oder einer schwefelsauren Lösung durchgeführt.

Wird in Gegenwart eines organischen Lösungsmittels gearbeitet, so kommen alle üblichen organischen Lösungsmittel in Frage, wie insbesondere Dimethylformamid.

Die Reaktionstemperaturen können bei der Durchführung der zweiten Stufe des erfindungsgemäßen Verfahrens in einem größeren Bereich variiert werden. Im allgemeinen arbeitet man zwischen 0 und 120°C, vorzugsweise zwischen 0 und 100°C.

Bei der Durchführung der zweiten Stufe des erfindungsgemäßen Verfahrens setzt man die Ausgangsstoffe vorzugsweise in molaren Mengen bzw. mit einem Überschuß an Thiocarbohydrazid der Formel (IV) ein. Die Isolierung der Zwischenprodukte der Formel (V) erfolgt in üblicher Weise.

Die dritte Stufe des erfindungsgemäßen Verfahrens wird in Gegenwart einer Base durchgeführt. Hierbei werden vorzugsweise Alkalihydroxide, wie Natriumhydroxid, in wäßriger Lösung oder Alkalialkoholate, wie Natriummethylat, wobei überschüssiger Alkohol als Lösungsmittel verwendet wird, eingesetzt.

Die Reaktionstemperaturen können bei der Durchführung der dritten Stufe des erfindungsgemäßen Verfahrens in einem größeren Bereich variiert werden. Im allgemeinen arbeitet man zwischen 0 und 100°C, vorzugsweise zwischen 0 und 50°C.
Bei der Durchführung der dritten Stufe des erfindungsgemäßen Verfahrens setzt man auf 1 Mol Zwischenprodukt der Formel (V) vorzugsweise 1 bis 1,5 Mol Alkylierungsmittel ein. Die Isolierung der Zwischenprodukte bzw. Endprodukte der Formel (Ia) erfolgt in üblicher Weise.

Als Verdünnungsmittel kommen für die vierte Stufe des erfindungsgemäßen Verfahrens vorzugsweise organische Lösungsmittel in Frage, wie insbesondere Isopropanol/Eisessig.

Die Reaktionstemperaturen können bei der Durchführung der vierten Stufe des erfindungsgemäßen Verfahrens in einem größeren Bereich variiert werden. Im allgemeinen arbeitet man zwischen 20 und 180°C, vorzugsweise zwischen 40 und 150°C.

Bei der Durchführung der vierten Stufe des erfindungsgemäßen Verfahrens setzt man auf 1 Mol der Verbindung der Formel (Ia) vorzugsweise 1 bis 3 Mol Amin der Formel (VI) ein. Die Isolierung der Endprodukte erfolgt in üblicher Weise.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

<u>Dikotyle Unkräuter der Gattungen:</u> Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst-, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe zeigen neben einer sehr guten allgemeinen herbiziden Wirkung eine gute Verträglichkeit gegenüber Nutzpflanzen. So ist es möglich, wichtige Schadgräser selektiv in wichtigen Kulturpflanzen wie z.B. Mais, Hafer und Weizen zu bekämpfen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, wirkstoffimprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle,

Alkohole, wie Butanol oder Glycol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z. B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen infrage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen infrage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion oder N-(2-Benzthiazolyl)-N,N'-dimethyl-harnstoff zur Unkrautbekämpfung in Getreide in Frage. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und

Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,05 und 10 kg Wirkstoff pro Hektar Bodenfläche, vorzugsweise zwischen 0,1 und 5 kg pro ha.

Die nachfolgenden Beispiele dienen zur weiteren Erläuterung der Erfindung.

Herstellungsbeispiele :

Beispiel 1 :

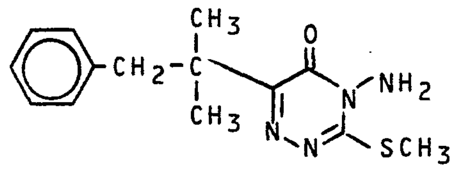

(1)

(1. und 2. Stufe)

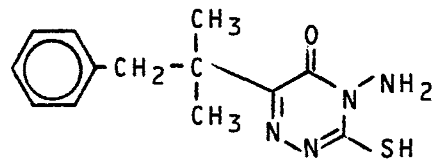

5,18 g (0,277 Mol) Phenylpivaloylcyanid werden bei Raumtemperatur zu 221 g Bromwasserstoff in Eisessig gegeben. Man läßt 2 Stunden bei Raumtemperatur nachrühren und versetzt dann unter Kühlung mit 5 ml Wasser, so daß die Innentemperatur 28°C nicht übersteigt. Man läßt wiederum 2 Stunden bei Raumtemperatur nachrühren und gibt dann 32,3 g (0,305 Mol) Thiocarbohydrazid in 305 ml 1n Salzsäure zu, wobei die Innentemperatur durch Kühlung auf ca. 26°C gehalten wird. Man läßt über Nacht bei Raumtemperatur nachrühren. Der ausgefallene Niederschlag wird abgesaugt und getrocknet.

Man erhält 45,2 g (59 % der Theorie) 4-Amino-3-mercapto-6-phenyl-tert.-butyl-1,2,4-triazin-5-on vom Schmelzpunkt 152 °C bis 155 °C.

(3. Stufe)

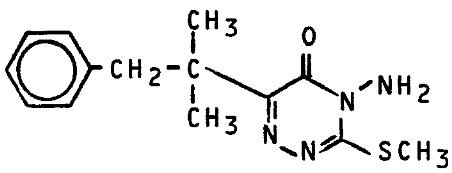

(1)

27,6 g (0,1 Mol) 4-Amino-3-mercapto-6-phenyl-tert.-butyl-1,2,4-triazin-5-on (gemäß Stufe 2) werden in 110 ml 1n Natronlauge gelöst. Nach vollständiger Lösung werden bei Raumtemperatur 15,6 g Methyljodid zugetropft. Nach beendeter Zugabe läßt man über Nacht bei Raumtemperatur nachrühren. Danach wird der entstehende Feststoff abgesaugt, mit Wasser gewaschen, getrocknet und aus n-Hexan umkristallisiert.

Man erhält 12,1 g (41,7 % der Theorie) 4-Amino-3-methylthio-6-phenyl-tert.-butyl-1,2,4-triazin-5-on vom Schmelzpunkt 155°C bis 157°C.

Herstellung des Ausgangsproduktes :

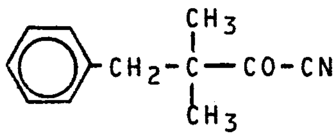

69,1 g (0,35 Mol) Phenylpivaloylchlorid werden auf 120°C bis 130°C erwärmt. Bei dieser Temperatur tropft man innerhalb von 40 Minuten 35 g (0,354 Mol) Trimethylsilylcyanid zu. Gleichzeitig wird das entstehende Trimethylsilylchlorid destillativ aus der Reaktionsmischung entfernt. Man läßt 20 Minuten bei 150°C nachrühren und destilliert das Reaktionsprodukt.

Man erhält 53 g Phenylpivaloylcyanid vom Siedepunkt 131°C bis 135°C/18 mbar.

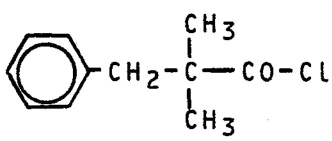

72 g (0,6 Mol) Thionylchlorid werden bei Raumtemperatur langsam zu 93 g (0,523 Mol) Benzyl-dimethyl-essigsäure getropft. Die Reaktionslösung wird anschließend ca. 4 Stunden bei 70°C nachgerührt, bis die Gasentwicklung beendet ist. Die Reaktionslösung wird fraktioniert.

Man erhält 70,6 g (68,5 % der Theorie) Phenylpivaloylchlorid vom Siedepunkt 118°C/10 mbar.

Beispiel 2 :

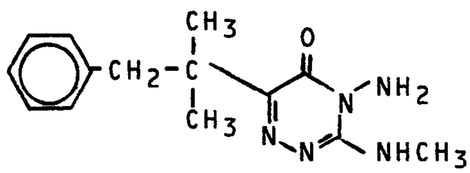

(2)

In eine Lösung von 6 g (0,1 Mol) Eisessig in 150 ml Isopropanol werden bei 5°C bis 10°C 0,2 Mol Methylamin eingeleitet. Diese Reaktionslösung wird anschließend mit 7,5 g (0,026 Mol) 4-Amino-3-methylthio-6-phenyl-tert.-butyl-1,2,4-triazin-5-on (Herstellung gemäß Beispiel 1) versetzt. Man läßt auf Raumtemperatur erwärmen und erhitzt anschließend ca. 14 Stunden unter Rückfluß. Danach wird das Reaktionsgemisch eingeengt und der ölige Rückstand in Wasser eingerührt. Man extrahiert mit Methylenchlorid, wäscht mit verdünnter Natronlauge und Wasser, trocknet über Natriumsulfat und engt ein. Der Rückstand wird in Essigester mit Aktivkohle umkristallisiert.

Man erhält 4,1 g (58 % der Theorie) 4-Amino-3-methylamino-6-phenyl-tert.-butyl-1,2,4-triazin-5-on vom Schmelzpunkt 195°C bis 196°C.

Beispiel 3 :

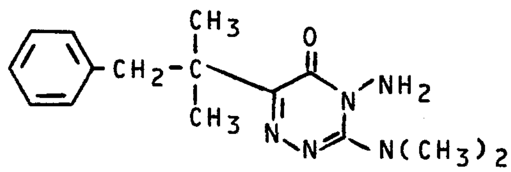

(3)

In eine Lösung von 6 g (0,1 Mol) Eisessig in 150 ml Isopropanol werden bei 5°C bis 10°C 0,2 Mol Dimethylamin eingeleitet. Diese Reaktionslösung wird anschließend mit 7,5 g (0,026 Mol) 4-Amino-3-methylthio-6-phenyl-tert.-butyl-1,2,4-triazin-5-on (Herstellung gemäß Beispiel 1) versetzt. Man läßt auf Raumtemperatur erwärmen und erhitzt anschließend ca. 14 Stunden unter Rückfluß. Danach wird das Reaktionsgemisch eingeengt und der Rückstand in Methylenchlorid aufgenommen. Man extrahiert mit 1n Salzsäure, stellt auf pH 14 und extrahiert mit Methylenchlorid. Die organische Phase wird über Natriumsulfat getrocknet und eingeengt. Der ölige Rückstand wird in n-Hexan zur Kristallisation gebracht.

Man erhält 4,6 g (62 % der Theorie) 4-Amino-3-dimethylamino-6-phenyl-tert.-butyl-1,2,4-triazin-5-on vom Schmelzpunkt 102°C bis 104°C.

In analoger Weise und entsprechend dem erfindungsgemäßen Verfahren werden die in der nachfolgenden Tabelle aufgeführten Verbindungen der Formel (I) erhalten :

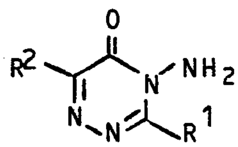

(I)

| Beisp.-Nr. | $R^1$ | $R^2$ | Schmelzpunkt (°C) bzw. $n_D^{20}$ |
|---|---|---|---|
| 4 | $-SCH_3$ | 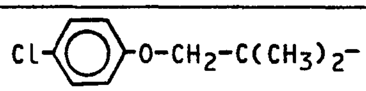 | 78 - 82 |
| 5 | $-N(CH_3)_2$ | 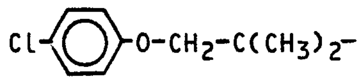 | 1,580 |
| 6 | $-SCH_3$ | $CH_3CO-O-CH_2-C(CH_3)_2-$ | 111 - 113 |
| 7 | $-SCH_3$ | $CH_3O-CH_2-C(C_2H_5)CH_3-$ | 1,554 |
| 8 | $-SCH_3$ | $C_2H_5O-CH_2-C(C_2H_5)CH_3-$ | 1,540 |
| 9 | $-N(CH_3)_2$ | $C_2H_5O-CH_2-C(C_2H_5)CH_3-$ | 1,537 |
| 10 | $-SCH_3$ | $Cl_2CH-C(CH_3)_2-$ | 173 - 175 |

Verwendungsbeispiele

In dem nachfolgenden Verwendungsbeispiel wird die nachstehend angegebene Verbindung als Vergleichssubstanz eingesetzt:

(A) 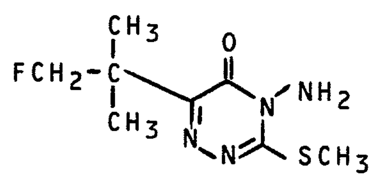

4-Amino-6-fluor-tert.-butyl-3-methylthio-1,2,4-triazin-5-on (bekannt aus EP-A-0 049 416).

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

In diesem Test zeigen z.B. die Verbindungen gemäß den Herstellungsbeispielen (2), (3) und (10) neben einer sehr guten allgemeinen herbiziden Wirksamkeit eine bessere Selektivität in Hafer, Weizen und Mais als die aus dem Stand der Technik bekannte Verbindung (A).

Patentansprüche

1. 3,6-Disubstituierte 4-Amino-1,2,4-triazin-5-one der allgemeinen Formel (I)

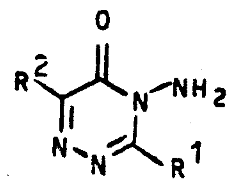

(I)

in welcher

$R^1$ für Alkylthio, Alkylamino oder Dialkylamino steht und

$R^2$ für die Gruppierungen

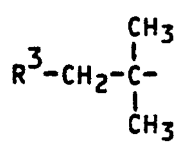 ; 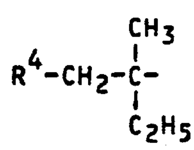 und 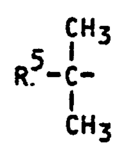 steht, wobei

$R^3$ für gegebenenfalls substituiertes Phenyl, gegebenenfalls substituiertes Phenoxy oder Alkylcarbonyloxy steht,

$R^4$ für Alkoxy steht und

$R^5$ für Dihalogenmethyl steht.

2. 4-Amino-3-methylamino-6-(phenyl-tert.-butyl)-1,2,4-triazin-5-on der Formel

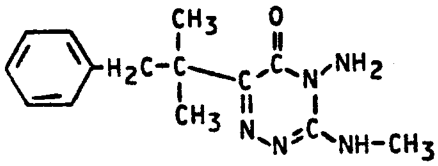

(2)

gemäß Anspruch 1.

3. 4-Amino-3-dimethylamino-6-(phenyl-tert.-butyl)-1,2,4-triazin-5-on der Formel

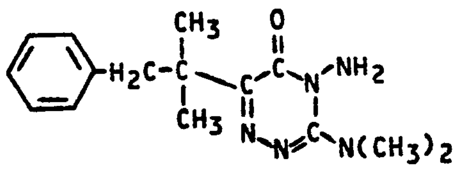

(3)

gemäß Anspruch 1.

4. 4-Amino-3-methylthio-6-(chlor-tert.-butyl)-1,2,4-triazin-5-on der Formel

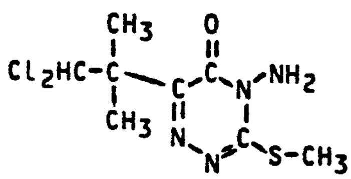

(10)

gemäß Anspruch 1.

5. Verfahren zur Herstellung von 3,6-disubstituierten 4-Amino-1,2,4-triazin-5-onen der allgemeinen Formel (I) gemäß Anspruch 1, dadurch gekennzeichnet, daß man in einer ersten Stufe Cyanide der Formel (II)

$$R^2\text{-CO-CN} \qquad \text{(II)}$$

in welcher

$R^2$ die oben angegebene Bedeutung hat,

(a) gegebenenfalls in Gegenwart einer flüssigen Carbonsäure als Lösungsmittel, mit einer anorganischen Säure umsetzt oder

(b) an ein Carboniumion addiert, das aus einem Olefin oder einem tertiären Alkohol mit einer starken Säure gebildet wird, und anschließend der Hydrolyse unterwirft (sog. RITTER-REAKTION)

und

die herbei entstehenden Ketocarbonsäureamide der Formel (IIIa)

$$R^2\text{-CO-CO-NHR} \qquad \text{(IIIa)}$$

in welcher

$R^2$ die oben angegebene Bedeutung hat und

R für Wasserstoff (erhältlich nach Variante (a)) oder Alkyl (erhältlich nach Variante (b)) steht,

in einer <u>zweiten Stufe</u> in an sich bekannter Weise entweder direkt in der anfallenden Lösung oder nach Zwischenisolierung, gegebenenfalls nach vorheriger Verseifung zu den freien Ketocarbonsäuren der Formel (IIIb)

$$R^2\text{-CO-COOH} \qquad \text{(IIIb)}$$

in welcher

$R^2$ die oben angegebene Bedeutung hat,

mit Thiocarbohydrazid der Formel (IV)

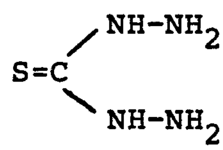

(IV)

in wäßriger bzw. wäßrig-saurer Lösung, gegebenenfalls in Gegenwart eines organischen Verdünnungsmittels, zu 4-Amino-3-mercapto-1,2,4-triazin-5-onen der Formel (V),

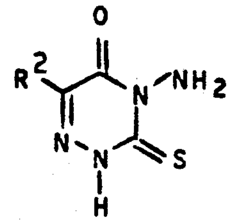

(V)

in welcher

$R^2$ die oben angegebene Bedeutung hat,

umsetzt; diese in einer dritten Stufe in bekannter Weise in alkalischer Lösung mittels Alkylhalogenid, vorzugsweise Alkyljodid oder -bromid, zu 3-Alkylthio-4-amino-1,2,4-triazin-5-onen der Formel (Ia)

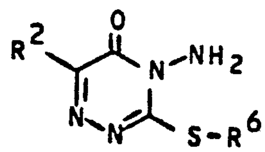

(Ia)

in welcher

$R^2$ die oben angegebene Bedeutung hat und

$R^6$ für Alkyl, vorzugsweise mit 1 bis 4 Kohlenstoffatomen, steht,

alkyliert, diese gegebenenfalls in einer vierten Stufe mit Aminen der Formel (VI)

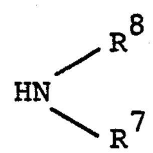

(VI)

in welcher

$R^8$ für Wasserstoff oder Alkyl, vorzugsweise mit 1 bis 6 Kohlenstoffatomen, steht und

$R^7$ für Alkyl, vorzugsweise mit 1 bis 6 Kohlenstoffatomen, steht,

in Gegenwart eines Verdünnungsmittels umsetzt.

6. Herbizide Mittel, gekennzeichnet durch einen Gehalt an mindestens einem 3,6-disubstituierten 4-Amino-1,2,4-triazin-5-on der Formel (I) gemäß Anspruch 1.

7. Verwendung von 3,6-disubstituierten 4-Amino-1,2,4-triazin-5-onen der Formel (I) gemäß Anspruch 1 zur Bekämpfung von unerwünschtem Pflanzenwachstum.

8. Verfahren zur Herstellung von herbiziden Mitteln, dadurch gekennzeichnet, daß man 3,6-disubstituierte 4-Amino-1,2,4-triazin-5-one der Formel (I) gemäß Anspruch 1 mit Streckmitteln und/oder oferflächenaktiven Mitteln vermischt.